# EUROPÄISCHE PATENTSCHRIFT

(11) **EP 1 009 375 B1**
(45) Veröffentlichungstag und Bekanntmachung des Hinweises auf die Patenterteilung: **27.02.2002**
(21) Anmeldenummer: 98948895.2
(22) Anmeldetag: 02.09.1998
(51) Int. Cl.: A61K 7/42

(54) **EMULGATORFREIES KLARES SONNENSCHUTZGEL**
EMULSIFIER-FREE TRANSPARENT SUN PROTECTION GEL
GEL DE PROTECTION SOLAIRE TRANSPARENT, SANS EMULSIFIANT

(30) Priorität: 02.09.1997 DE 19739447
(43) Veröffentlichungstag der Anmeldung: 21.06.2000
(73) Patentinhaber: Coty B.V., 2031 CC Haarlem (NL)
(72) Erfinder: CERNASOV, Domnica, Ringwood, NJ 07456 (US); SYGENGCO, Noel, Franklyn Park, NJ 08823 (US); MACCHIO, Ralph, Flanders, NJ 07836 (US); KULKARNI, Rupali, Bridgewater, NJ 08807 (US)
(74) Vertreter: Walter, Wolf-Jürgen
(86) Internationale Anmeldenummer: EP9805556
(87) Internationale Veröffentlichungsnummer: WO9911236

(56) Entgegenhaltungen:
- WO-A-93/02117
- FR-A- 1 256 438
- US-A- 3 821 363

## Beschreibung

Die Erfindung betrifft ein neues klares Sonnenschutzgel, das ohne Emulgatorgehalt eine ausgezeichnete Stabilität und hohe Gehalte an Öl bzw. Lichtschutzmitteln aufweist.

Aus der DE 43 03 983 sind kosmetische Lichtschutzformulierungen bekannt, die auf Basis einer Hydrodispersion anorganische Pigmente und organische UVA- und UVB-Filtersubstanzen enthalten können und dabei ohne Emulgator zu nichtklebrigen und nicht agglomerierenden Zubereitungen verarbeitet werden können.

Weiterhin sind aus der US-A-5035890 emulgatorfreie Gele als Hand- und Bodylotionen bekannt. Die in den Beispielen aufgeführten hohen Ölgehalte sind jedoch auf das Roh-Gemisch beschränkt, und bei Wassergehalten von > 1 Gew-%, bezogen auf das Endprodukt, erhält man ein milchig-trübes Gel.

Der Erfindung liegt die Aufgabe zugrunde, neue klare Gele bereitzustellen, die auf Basis hoher ölgehalte auch hohe Anteile an organischen Lichtschutzmitteln enthalten können.

Die Aufgabe wird erfindungsgemäß gelöst durch ein emulgatorfreies klares Sonnenschutzgel, das gekennzeichnet ist durch einen Gehalt an
a) einem mit Decadien vernetzten copolymeren von Methylvinylether und Maleinsäure im Bereich von 0,01 bis 15 Gew-%;
b) einem kosmetisch einsetzbaren öl im Bereich von 0,1 bis 30 Gew-%;
c) einem organischen Lichtschutzmittel oder Lichtschutzmittelgemisch im Bereich von 0,1 bis 30 Gew-%;
d) Wasser im Bereich von >1 bis 85 Gew-%;
e) kosmetisch üblichen Additiven im Bereich von 1 bis 50 Gew-%, wobei der Gehalt an Lichtschutzmitteln und anderen Additiven zusammen im Bereich von 1,1 bis 45 Gew-% liegt;
und wobei alle Prozentangaben jeweils auf den Gehalt der Gesamtzusammensetzung bezogen sind;
und das hergestellt ist durch Vermischen des mit Decadien vernetzten Copolymeren von Methylvinylether und Maleinsäure mit Wasser unter Temperaturerhöhung auf 70-80 °C zu einem vorgeformten Gel und Einbringen der Lichtschutzmittel ohne Wasserphase direkt in das vorgeformte Gel unter Rühren bei 30-40 °C bis zur Bildung des klaren Gels, wobei das vorgeformte Gel gegebenenfalls weitere Bestandteile der Formulierung enthalten kann.

Das Copolymere ist nicht-acrylisch und benzenfrei und beispielsweise unter dem CTFA-Namen "PVM/MA Decadiene Crosspolymer" als Stabilisator bekannt.

Das Copolymere wird in dem erfindungsgemäßen Sonnenschutzgel vorzugsweise in einer Menge von 0,5 bis 10 Gew-%, insbesondere 1 bis 5 Gew-% eingesetzt.

überraschenderweise lassen sich mit dem Copolymeren klare halbfeste Gele bilden auch mit Gehalten an öl und sonstigen Additiven zusammen bis zu 45 Gew-%, teilweise bis 50 Gew-% und sogar bis 55 Gew-%. Dadurch wird es möglich, größere Mengen an organischen Sonnenschutzfiltern in das Gel aufzunehmen und damit Lichtschutzfaktoren (SPF = sun protection factor) im Bereich von SPF 15 - 20 zu erreichen. Die Bildung klarer d.h. transparenter Gele ist insbesondere auch deshalb überraschend, weil Wassergehalte über 1 Gew-% zusammen mit den organischen Lichtschutzmitteln und den ölen normalerweise zu milchigen Emulsionen und damit milchigen oder trüben Gelen führen. Durch die erfindungsgemäße Verfahrensweise wird jedoch auch bei hohen Wassergehalten zusammen mit den ölen und Lichtschutzmitteln der Formulierung ein klares Gel erhalten.
Als Öle können erfindungsgemäß flüssige Ester wie Triglyceride, Dicaprylmaleat oder Isopropylmyristat, Mineralöl, pflanzliche Öle, Siliconöle wie Dimethicone und Cyclomethicone usw. sowie Gemische davon eingesetzt werden. Besonders bevorzugt sind Siliconöle.

Bevorzugte Bereiche für die öligen Substanzen sind 0,5 bis 25 Gew-%, insbesondere 1 bis 15 Gew-%.

Als organische Lichtschutzmittel können eingesetzt werden Benzophenon-Derivate, 3-Benzylidencampher-Derivate, Ester der Zimtsäure oder der Salicylsäure, 4-Aminobenzoesäure-Derivate, wie zum Beispiel Benzophenone-3, Butylmethoxydibenzoylmethane, Octylmethoxycinnamate, Octylsalicylate sowie auch wasserlösliche Produkte wie Phenylbenimidazolsulfonsäure oder Benzophenonsulfonsäure-Derivate. Auch Kombinationen mit UVA-Filtern sind möglich.

Die Menge an Lichtschutzmittel allein liegt vorzugsweise im Bereich von 5 bis 20 Gew-%, insbesondere 5 bis 15 Gew-%; zusammenmit anderen Additiven liegt sie vorzugsweise zusammen im Bereich von 10 bis 50 Gew-%.

Als kosmetisch nützliche Additive können eingesetzt werden Alkohole, Diole, Polyole, wie zum Beispiel Ethanol, Isopropanol, Propylenglycol, Glycerin, Ethylenglycol, C₉-C₁₁-Alkohole usw.; Methyl-, Ethyl oder Butylether mit Ethylenglycol, Propylenglycol oder Diethylenglycol; Vitamine wie Vitamin A oder Vitamin E; Panthenol, Aloe vera, Allantoin, Bisabolol; Farbstoffe; Konservierungsmittel; Schutzmittel; Feuchthaltemittel; Antioxidationsmittel; pH-Regulatoren; Pigmente wie lösliches Melanin; Parfüm; Kupfergluconat usw.

Dabei kann der Gehalt der Additive allein vorzugsweise im Bereich von 1 bis 30 Gew-% liegen, zusammen mit Sonnenschutzmitteln jedoch bis zu 55 Gew-%. Ein solch hoher Gehalt in einem klaren Gel läßt besonders gute Gestaltungsmöglichkeiten für spezielle Anwendungsformen des Gels zu. hoher Gehalt in einem klaren Gel läßt besonders gute Gestaltungsmöglichkeiten für spezielle Anwendungsformen des Gels zu.

Ein besonderer Wasseranteil beträgt 10 bis 85 Gew-%.

Ein bevorzugtes Sonnenschutzgel enthält:
(a) mit Decadien vernetzte Copolymere von Methylvinylether und Maleinsäure im Bereich von > 0,1 bis 5 Gew-%;
(b) ein kosmetisch einsetzbares öl oder ölgemisch und (c) ein organisches Sonnenschutzmittel zusammen im Bereich von 9 bis 18 Gew-%;
(d) Wasser im Bereich von 40 bis 85 Gew-%; und vorzugsweise (e) kosmetisch übliche Additive im Bereich von 1 bis 30 Gew-%, wobei der Gehalt an Sonnenschutzmitteln (c) und anderen Additiven (e) zusammen im Bereich von bis zu 55 Gew-% liegt.

Wasser kann in einer Menge von mehr als 1 Gew-% bis 85 Gew-% vorhanden sein, insbesondere 40 bis 85 Gew-%. Einen Emulgator enthält das erfindungsgemäße Gel nicht.

Ein besonders vorteilhafte klares Sonenschutzgel enthält:
mit Decadien vernetzte Copolymere von Methylvinylether und Maleinsäure im Bereich von > 0,1 bis 5 Gew-%;
ein kosmetisch einsetzbares öl oder ölgemisch und ein organisches Lichtschutzmittel zusammen im Bereich von 5 bis 18 Gew-%;
Wasser im Bereich von 40 bis 85 Gew-%;
kosmetisch übliche Additive im Bereich von 5 bis 25 Gew-%, wobei der Gehalt an Lichtschutzmitteln und anderen Additiven zusammen im Bereich von 25 bis 40 Gew-% liegt.

Gegenüber den bereits genannten Vorteilen des erfindungsgemäßen Sonnenschutzmittels ist das gebildete Gel nicht klebrig, was bei üblichen Gelen mit höheren Wassergehalten normalerweise der Fall ist. Es ist weiterhin sehr glatt und samtartig und hat mehr das Aussehen eines Hautpflegeproduktes. Es ist nicht ölig, wie dies bei vergleichbaren Emulsionen der Fall sein kann. Infolge der Transparenz kann das Gel auch in besonders ästhetischen Verpakkungen wie transparenten Tuben, Druckbehältern etc. angeboten werden

Die Herstellung des erfindungsgemäßen klaren Gels erfolgt ohne Emulgator in der Weise, daß das vernetzte Coplymere als Pulver unter starkem Rühren und Homogenisieren in vorgelegtes Wasser eingesprüht wird und danach zwecks Hydratisierung des Copolymeren die Temperatur auf 70 bis 75 °C erhöht wird. Nach dem Abkühlen wird die ölphase mit den entsprechenden Bestandteilen zugesetzt und dann die Lichtschutzmittel in flüssiger Form und unter Rühren bei 30 bis 35 °C. Danach erfolgt die Einstellung der gewünschten Konsistenz (Viskosität) in bekannter Weise, zum Beispiel durch Neutralisation mit einer vorgemischten Aminlösung.

Das erfindungsgemäße Gel wurde über mehrere Monate stabil bei Raumtemperatur und bei 40 und 45 und 50 °C gelagert, ohne daß Veränderungen festgestellt wurden. Auch Tests mit Frost/Tau-Zyklen ergaben keine nachteiligen Werte. Die nach Brookfield gemessene Viskosität, die allgemein zwischen 13.000 und 17.000 cps lag, wurde über den Lagerzeitraum beibehalten.

Die Erfindung soll nachstehend durch Beispiele näher erläutert werden. Alle Angaben darin sind auf das Gewicht bezogen, wenn nichts anderes angegeben ist.

| Beispiel 1 **Transparentes Sonnenschutzgel SPF 8** | |
|---|---|
| **Phase A** | |
| Glycerin | 2,0 |
| Allantoin | 0,5 |
| PVM/MA Decadiene Crosspolymer | 0,53 |
| Disodium EDTA | 0,05 |
| deionisiertes Wasser | q.s. ad 100 |

| **Phase B** | |
|---|---|
| Propylenglycol/Wasser/Glyceryl | |
| Polymethacrylate | 3,0 |

| **Phase C** | |
|---|---|
| lösliches Melanin | 0,02 |
| D-Panthenol | 1,0 |
| Wasser | 3,0 |

| **Phase D** | |
|---|---|
| Kupfergluconat | 0,1 |
| Aloe vera | 0,1 |
| Glycine | 0,05 |
| Wasser | 2,0 |

| **Phase E** | |
|---|---|
| Farbstoffe | 2,0 |

| **Phase F** | |
|---|---|
| Dimethicone | 1,0 |

| **Phase G** | |
|---|---|
| Octyl Methoxycinnamate | 7,0 |
| Benzophenone-3 | 2,0 |
| Butyl Methoxydibenzoylmethane | 1,0 |
| Tocopheryl Acetate | 0,2 |

| **Phase H** | |
|---|---|
| Parfüm | 0,3 |

| **Phase I** | |
|---|---|
| Konservierungsmittel | 0,6 |

| **Phase J** | |
|---|---|
| Tromethamine | 0,4 |
| Wasser | 2,0 |

| Beispiel 2 **Transparentes Sonnenschutzgel II - SPF 15** | |
|---|---|
| **Phase A** | |
| Allantoin | 1,0 |
| PVM/MA Decadiene Crosspolymer | 0,2 |
| Disodium EDTA | 0,1 |
| deionisiertes Wasser | q.s. ad 100 |

| **Phase B** | |
|---|---|
| Propylenglycol/Wasser/Glyceryl | |
| Polymethacrylate | 2,0 |

| **Phase C** | |
|---|---|
| lösliches Melanin | 0,1 |
| D-Panthenol | 2,0 |
| Wasser | 3,0 |

| **Phase D** | |
|---|---|
| Aloe vera | 0,2 |
| Wasser | 2,0 |

| Phase E | |
|---|---|
| Farbstoffe | 4,0 |

| **Phase F** | |
|---|---|
| Dimethicone | 2,0 |

| **Phase G** | |
|---|---|
| Octyl Methoxycinnamate | 7,5 |
| Benzophenone-3 | 3,0 |
| Butyl Methoxydibenzoylmethane | 2,0 |
| Tocopheryl Acetate | 2,0 |

| **Phase H** | |
|---|---|
| Parfüm | 0,6 |

| **Phase I** | |
|---|---|
| Konservierungsmittel | 0,7 |

| **Phase J** | |
|---|---|
| Tromethamine | 0,5 |
| Wasser | 2,0 |

| Beispiel 3 **Transparentes Sonnenschutzgel III - SPF 18** | |
|---|---|
| **Phase A** | |
| Glycerin | 5,0 |
| Allantoin | 2,0 |
| PVM/MA Decadiene Crosspolymer | 1,5 |
| Disodium EDTA | 0,2 |
| deionisiertes Wasser | q.s. ad 100 |

| **Phase B** | |
|---|---|
| Propylenglycol/Wasser/Glyceryl | |
| Polymethacrylate | 5,0 |

| **Phase C** | |
|---|---|
| lösliches Melanin | 0,2 |
| D-Panthenol | 3,0 |
| Wasser | 3,0 |

| **Phase D** | |
|---|---|
| Kupfergluconat | 0,1 |
| Aloe vera | 0,5 |
| Glycine | 0,08 |
| Wasser | 2,0 |

| **Phase E** | |
|---|---|
| Farbstoffe | 6,0 |

| **Phase F** | |
|---|---|
| Dimethicone | 3,0 |

| **Phase G** | |
|---|---|
| Octyl Methoxycinnamate | 7,5 |
| Benzophenone-3 | 4,0 |
| Butyl Methoxydibenzoylmethane | 4,0 |
| Tocopheryl Acetate | 1,0 |

| **Phase H** | |
|---|---|
| Parfüm | 0,4 |

| **Phase I** | |
|---|---|
| Konservierungsmittel | 0,9 |

| **Phase J** | |
|---|---|
| Tromethamine | 0,6 |
| Wasser | 3,0 |

Die Bestandteile der Phase A wurden jeweils vermischt unter Einsprühen von pulverförmigem PVM/MA Decadiene Crosspolymer, und die Temperatur wurde auf etwa 75 °C unter Rühren erhöht. Bei etwa 70 °C wurde die Phase B hinzugegeben und dann auf 30 bis 35 °C abgekühlt. Bei dieser Temperatur wurde die separat vorgemischte Phase C hinzugegeben und dann die ebenfalls vorgemischte Phase D und die Farbstoffe der Phase E.

Nach Zugabe der Phase F bis zur Verteilung der gebildeten feinen Tröpfchen wurden die Bestandteile der Phase G separat bei 50 bis 55 °C gemischt, auf 30 bis 35 °C abgekühlt und dann dem Gemisch im Hauptbehälter zugesetzt.

Nacheinander wurden anschließend die Phasen H, I und J zugegeben und bei 25 bis 30 °C bis zum Erhalt eines klaren Gels vermischt.

Für das erhaltenen Produkt wurde der SPF in vivo unter Bestrahlung mit einer 150 W Xenon-Bogenlampe gemäß "Proposed Monograph for OTC Sunscreen Drug Products" der US-Food and Drug Administration v. 25.8.78, Fed. Reg. Vol 43, No. 166, 38206-38269 gemessen.

## Patentansprüche

1. Emulgatorfreies klares Sonnenschutzgel, **gekennzeichnet durch** einen Gehalt an
(a) einem mit Decadien vernetzten Copolymeren von Methylvinylether und Maleinsäure im Bereich von 0,01 bis 15 Gew-%;
(b) einem kosmetisch einsetzbaren Öl im Bereich von 0,1 bis 30 Gew-%;
(c) einem organischen Lichtschutzmittel oder Lichtschutzmittelgemisch im Bereich von 0,1 bis 30 Gew-%;
(d) Wasser im Bereich von >1 bis 85 Gew-%;
(e) kosmetisch üblichen Additiven im Bereich von 1 bis 50 Gew-%,
wobei der Gehalt an Lichtschutzmitteln (c) und anderen Additiven (e) zusammen im Bereich von 1,1 bis 55 Gew-% liegt;
und wobei alle Prozentangaben jeweils auf den Gehalt der Gesamtzusammensetzung bezogen sind;
und das hergestellt ist **durch** Vermischen des mit Decadien vernetzten Copolymeren von Methylvinylether und Maleinsäure mit Wasser unter Temperaturerhöhung auf 70-80 °C zu einem vorgeformten Gel und Einbringen der Lichtschutzmittel ohne Wasserphase direkt in das vorgeformte Gel unter Rühren bei 30-40 °C bis zur Bildung des klaren Gels, wobei das vorgeformte Gel gegebenenfalls weitere Bestandteile der Formulierung enthalten kann.

2. Sonnenschutzgel nach Anspruch 1, **dadurch gekennzeichnet, daß** das Copolymere von Methylvinylether und Maleinsäure im Bereich von 0,5 bis 10 Gew-% enthalten ist.

3. Sonnenschutzgel nach Anspruch 2, **dadurch gekennzeichnet, daß** das Copolymere von Methylvinylether und Maleinsäure im Bereich von 0,5 bis 5 Gew-% enthalten ist.

4. Sonnenschutzgel nach Anspruch 1, **dadurch gekennzeichnet, daß** das Öl im Bereich von 0,1 bis 25 Gew-% enthalten ist.

5. Sonnenschutzgel nach Anspruch 4, **dadurch gekennzeichnet, daß** das Öl im Bereich von 0,1 bis 15 Gew-% enthalten ist.

6. Sonnenschutzgel nach Anspruch 1, **dadurch gekennzeichnet, daß** das Sonnenschutzmittel im Bereich von 5 bis 20 Gew-% enthalten ist.

7. Sonnenschutzgel nach Anspruch 1, **dadurch gekennzeichnet, daß** Wasser im Bereich von 10 bis 85 Gew-% enthalten ist.

8. Sonnenschutzgel nach Anspruch 7, **dadurch gekennzeichnet, daß** Wasser im Bereich von 40 bis 85 Gew-% enthalten ist.

9. Sonnenschutzgel nach Anspruch 1, **dadurch gekennzeichnet, daß** der Gehalt an Sonnenschutzmitteln und anderen Additiven zusammen im Bereich von 10 bis 50 Gew-% liegt.

10. Sonnenschutzgel nach Anspruch 1, **dadurch gekennzeichnet, daß** das Öl ein flüssiger Ester, ein Mineralöl, ein Pflanzenöl, ein Siliconöl oder ein Gemisch davon ist.

11. Sonnenschutzgel nach Anspruch 1, **dadurch gekennzeichnet, daß** es enthält:
(a) mit Decadien vernetzte Copolymere von Methylvinylether und Maleinsäure im Bereich von > 0,1 bis 5 Gew-%;
(b) ein kosmetisch einsetzbares Öl oder Ölgemisch und (c) ein organisches Sonnenschutzmittel zusammen im Bereich von 9 bis 18 Gew-%;
(d) Wasser im Bereich von 40 bis 85 Gew-%; und vorzugsweise (e) kosmetisch übliche Additive im Bereich von 1 bis 30 Gew-%, wobei der Gehalt an Sonnenschutzmitteln (c) und anderen Additiven (e) zusammen im Bereich von bis zu 55 Gew-% liegt.

12. Sonnenschutzgel nach Anspruch 1 oder 11, **dadurch gekennzeichnet, daß** das kosmetische Öl ein flüchtiges Öl ist.

## Claims

1. Emulsifier free transparent sun protection gel
**characterized by** a content of
a) a decadiene cross-linked copolymer of methyl vinyl ether and maleic acid in the range from 0.01 to 15 % by weight;
b) a cosmetically usable oil in the range from 0.1 to 30 % by weight;
c) an organic light protection agent in the range from 0.1 to 30 % by weight;
d) water in the range from > 1 to 85 % by weight;
e) conventional cosmetic additives in the range from 1 to 50 % by weight and wherein the content of light protection agents (c) and other additives (e) is in the range from 1.1 to 55 % by weight;
and wherein all percentages refer to the contents of the overall composition;
and which is produced by mixing the cross-linked copolymer of methylvinyl ether and maleic acid with water with raising the temperature to 70 to 80 °C to a preformed gel and introducing the light protection agent without any water phase directly into the preformed gel with stirring at 30 to 40 °C until forming the clear gel wherein the preformed gel optionally contains further components of the formulation.

2. Sun protection gel according to claim 1 wherein the copolymer of methyl vinyl ether and maleic acid is contained in the range from 0.5 to 10 % by weight.

3. Sun protection gel according to claim 2 wherein the copolymer of methyl vinyl ether and maleic acid is contained in the range from 0.5 to 5 % by weight.

4. Sun protection gel according to claim 1 wherein the oil is contained in the range from 0.1 to 25 % by weight.

5. Sun protection gel according to claim 4 wherein the oil is contained in the range from 0.1 to 15 % by weight.

6. Sun protection gel according to claim 1 wherein the light protection agent is contained in the range from 5 to 20 % by weight.

7. Sun protection gel according to claim 1 wherein the water is contained in the range from 10 to 85 % by weight.

8. Sun protection gel according to claim 1 wherein the water is contained in the range from 40 to 85 % by weight.

9. Sun protection gel according to claim 1 wherein the light protection agent and other additive contents together are in the range from 10 to 50 % by weight.

10. Sun protection gel according to claim 1 wherein the oil comprises a liquid ester, a mineral oil, a vegetable oil, a silicone oil or a mixture thereof.

11. Sun protection gel according to claim 1 **characterized by** a content of:
(a) copolymers of decadiene cross-linked methyl vinyl ether and maleic acid in the range from > 0.1 to 5 % by weight;
(b) a cosmetically usable oil or oil blend and (c) an organic sun protection agent together in the range from 9 to 18 % by weight;
(d) water in the range from 40 to 85 % by weight; and preferably (e) conventional cosmetic additives in the range from 1 to 30 % by weight wherein the content of sun protection agents (c) and other additives (e) together is in the range from up to 55 % by weight.

12. Sun protection gel according to claim 1 or 11 wherein the cosmetically oil is a volatile oil.

## Revendications

1. Gel de protection solaire transparent, sans émulsifiant, **caractérisé par** une teneur en :
(a) un copolymère d'éther méthylvinylique et d'acide maléique réticulé avec du décadiène, dans la plage allant de 0,1% à 15% en poids ;
(b) une huile cosmétiquement utilisable, dans la plage allant de 0,1 à 30% en poids ;
(c) un agent organique de protection contre la lumière ou un mélange d'agents organiques de protection contre la lumière, dans la plage allant de 0,1 à 30% en poids ;
(d) de l'eau, dans la plage allant de plus de 1% jusqu'à 85% en poids ;
e) des additifs cosmétiques classiques, dans la plage allant de 1 à 50% en poids, la teneur en agents de protection contre la lumière (c) et en autres additifs (e) se situant ensemble dans la plage allant de 1,1 à 55% en poids,
et toutes les pourcentages indiqués se rapportant respectivement à la teneur de la composition totale ;
et qui est fabriqué en mélangeant le copolymère d'éther méthylvinylique et d'acide maléique réticulé avec du décadiène avec de l'eau en augmentant la température à 70-80°C pour obtenir un gel préformé et en introduisant l'agent de protection contre la lumière sans phase aqueuse directement dans le gel préformé sous agitation à 30-40°C jusqu'à la formation d'un gel transparent, le gel préformé pouvant le cas échéant contenir d'autres composants de la préparation.

2. Gel de protection solaire selon la revendication 1, **caractérisé en ce que** le copolymère d'éther méthylvinylique et d'acide maléique est présent en une quantité dans la plage de 0,5% à 10% en poids.

3. Gel de protection solaire selon la revendication 2, **caractérisé en ce que** le copolymère d'éther méthylvinylique et d'acide maléique est présent en une quantité dans la plage de 0,5% à 5% en poids.

4. Gel de protection solaire selon la revendication 1, **caractérisé en ce que** l'huile est présente en une quantité dans la plage de 0,1% à 25% en poids.

5. Gel de protection solaire selon la revendication 4, **caractérisé en ce que** l'huile est présente en une quantité dans la plage de 0,1% à 15% en poids.

6. Gel de protection solaire selon la revendication 1, **caractérisé en ce que** l'huile est présente en une quantité dans la plage de 5% à 20% en poids.

7. Gel de protection solaire selon la revendication 1, **caractérisé en ce que** l'eau est présente en une quantité dans la plage de 10% à 85% en poids.

8. Gel de protection solaire selon la revendication 7, **caractérisé en ce que** l'eau est présente en une quantité dans la plage de 40% à 85% en poids.

9. Gel de protection solaire selon la revendication 1, **caractérisé en ce que** la teneur en agents de protection solaire et en autres additifs se situe ensemble dans la plage allant de 10% à 50% en poids.

10. Gel de protection solaire selon la revendication 1, **caractérisé en ce que** l'huile est un ester liquide, une huile minérale, une huile végétale, une huile de silicone ou un mélange de ceux-ci.

11. Gel de protection solaire selon la revendication 1, **caractérisé en ce qu'**il comprend :
(a) des copolymères d'éther méthylvinylique et d'acide maléique réticulé avec du décadiène, dans la plage allant de plus de 1% jusqu'à 5% en poids ;
(b) une huile ou un mélange d'huiles cosmétiquement utilisable et (c) un agent organique de protection solaire conjointement dans la plage allant de 9 à 18% en poids ;
(d) de l'eau, dans la plage allant de 40 à 85% en poids ; et, de préférence, (e) des additifs cosmétiques classiques dans la plage allant de 1 à 30% en poids, la teneur en agents de protection solaire (c) et en autres additifs (e) se situant ensemble dans la plage allant jusqu'à 55% en poids.

12. Gel de protection solaire selon la revendication 1 ou 11, **caractérisé en ce que** l'huile cosmétique est une huile volatile.
